# EUROPEAN PATENT APPLICATION

(11) **EP 0 695 557 A1**
(43) Date of publication of application: **07.02.1996**
(21) Application number: 95202023.8
(22) Date of filing: 21.07.1995
(51) Int. Cl.: A61M 25/10

(54) **Balloon catheter**

(30) Priority: 29.07.1994 NL 9401244
(71) Applicant: CORDIS EUROPA N.V., NL-9301 LJ Roden (NL)
(72) Inventor: Klunder, Rento Willem, NL-9742 EV Groningen (NL)
(74) Representative: 't Jong, Bastiaan Jacobus

(57) **Abstract**

The invention relates to a catheter comprising an outer tube-like element with a proximal and a distal end and an inflatable balloon body fitted close to the distal end to the outer tube-like element. The balloon body comprises a neck pushed over the end of the outer tube-like member and a balloon member connecting to the neck which, in a state of rest, is folded together in a number of folds flattened in the circumferential direction. An inner tube-like element extends through the outer tube-like element and the balloon body, to which the end of the balloon body opposite to the neck is connected. The neck comprises, in between the section pushed onto the outer tube-like body and the balloon member, a receding section with a smaller diameter than the section of the neck pushed onto the outer tube-like body.

## Description

The invention relates to a balloon catheter.

More in particular, the invention relates to a balloon catheter with a balloon of the type which, in a state of rest, has been folded together in a number of folds flattened in the circumferential direction. The balloon member comprises a neck which has been pushed partly over the end-section of an outer tube-like element of the catheter and has been fixed to it. An inner tube-like element extends through the outer tube-like element and the balloon. At the distal end the balloon member has been attached to this inner tube-like element.

When using such a balloon catheter it is inserted into a patient via the channel of an introduction member introduced beforehand.

With the development of catheters a tendency towards ever smaller effective diameters can be observed.

The object of the invention is to provide a catheter of the type as described in the preamble which has a smaller effective diameter, that is to say, which can be introduced through a channel with a smaller diameter.

This object is achieved with the catheter as characterised in claim 1.

It has become apparent that with a balloon catheter with a folded balloon, the section of the folded balloon which is adjacent to the neck, that is to say the section of the balloon where the folds start, is less flexible and is therefore folded together less well. This section therefore determines the effective diameter of the catheter. Because of the measure according to the invention the section of the balloon concerned starts from a smaller diameter, that is to say the smaller diameter of the receding section of the neck, as a result of which also the initial section of the folded balloon has a, to the same degree smaller diameter, so that also the effective diameter of the catheter has a smaller diameter.

The measure as set out in claim 2 is preferably employed. Because of this the effective cross-section of the channel which is formed between the outer tube-like element and the inner tube-like element is also at the receding section of the neck continuous, so that on the one hand there will be no obstruction to flow through this channel and on the other hand a maximal effect of the invention will be achieved. The purpose of this channel is to supply a fluid under pressure to the balloon in order to make the balloon inflate.

The invention will be explained in greater detail in the following description with reference to the attached drawings.

Figure 1 shows a partial view of a catheter according to the invention.

Figure 2 represents a perspective view of part of the catheter according to the invention, cut through according to line II-II in figure 1.

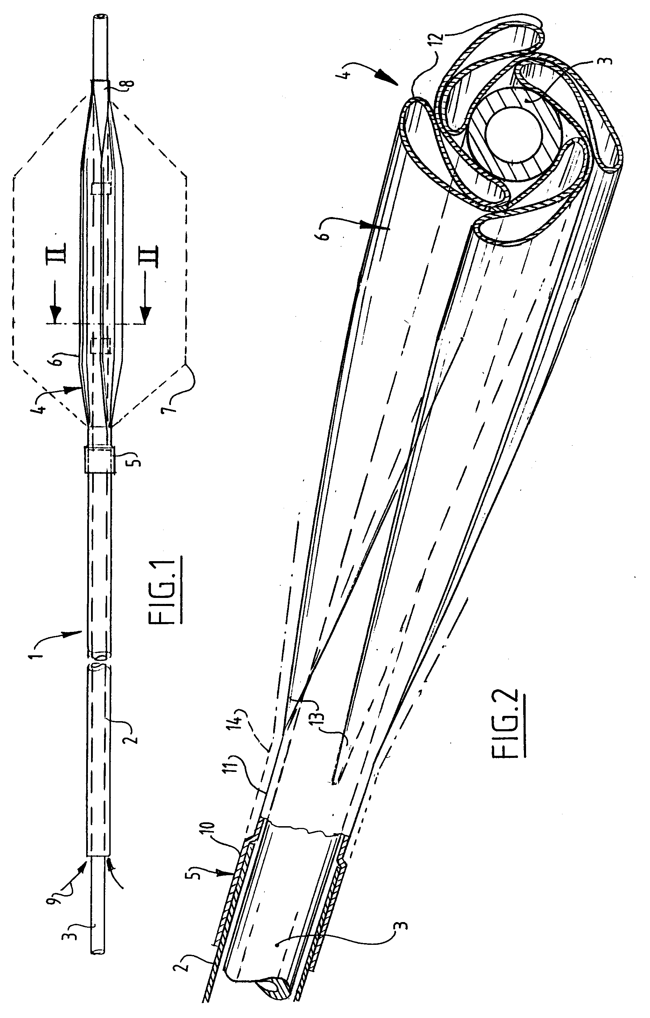

The catheter 1 shown in figure 1 comprises an outer tube-like element 2 with, as seen in figure 1, the distal end on the right hand side and the proximal end on the left hand side. A connecting member not shown here, is usually attached to the proximal end.

A balloon body 4 is attached to the distal end of the outer tube-like element 2. This balloon body 4 comprises a neck 5 which has been pushed over and attached to the end of the outer tube-like element 2 and a balloon member 6.

An inner tube-like element 3 extends through the outer tube-like element 2 and the balloon body 4. At the end 8, turned away from the neck 5, the balloon body 4 is attached to this inner tube-like element 3.

Thus a channel is formed between the inner tube-like element 3 and the outer tube-like element 2, which is connected with the inside of the balloon member 6 and is closed off at the distal end by means of the connection of the balloon body 4 to the inner tube-like element. By supplying medium under pressure as indicated by arrow 9, the balloon member 6 can be inflated to the shape indicated by the dotted lines 7.

The balloon member 6 is of a type which, in a state of rest, is folded together in a number of folds 12 flattened in the circumferential direction.

The material thickness of the balloon member 6 is, for instance at the cross-section II-II, very thin. Inside the folds the material fits snugly, as a result of which the effective diameter at that point is equal to the diameter of the inner tube-like element 3 plus a number of times the material thickness of the balloon member 6.

However, at the beginning of the folds 12 indicated with the number 13, the folds cannot lie completely against each other due to the relatively greater stiffness of the material at that point, as a result of which the effective diameter in the folded state is greatest there.

As is shown in particular in figure 2, the neck 5 of the balloon body 4 has been provided with a first section 10 which has been pushed over the outer tube-like element 2 and an adjacent receding section 11. As consequently the neck 5 has a smaller diameter at the beginning 13 of the folds 12, the effective diameter at the decisive point, that is to say at the beginning 13 of the folds 12, is proportionally smaller. The difference has been indicated schematically with the dashed and dotted line 14, which shows the diameter in the absence of a receding section 11.

## Claims

1. Catheter comprising an outer tube-like element with a proximal and a distal end, an inflatable balloon body fitted close to the distal end to the outer tube-like element which comprises a neck pushed over the end of the outer tube-like member and a balloon member connecting to the neck which, in a state of rest, is folded together in a number of folds flattened in the circumferential direction, wherein an inner tube-like element extends through the outer tube-like element and the balloon body, to which the end of the balloon body opposite to the neck is connected, and wherein the neck comprises, in between the section pushed onto the outer tube-like body and the balloon member, a receding section with a smaller diameter than the section of the neck pushed onto the outer tube-like body.

2. Catheter as claimed in claim 1, wherein the receding section of the neck has an inside diameter which is substantially equal to the inside diameter of the outer tube-like element.
